# EUROPÄISCHE PATENTSCHRIFT

(11) **EP 3 380 093 B1**
(45) Veröffentlichungstag und Bekanntmachung des Hinweises auf die Patenterteilung: **07.04.2021**
(21) Anmeldenummer: 16794515.3
(22) Anmeldetag: 02.11.2016
(51) Int. Cl.: A61K 31/12, A61P 17/02

(54) **BETA-HYDROXY-KETONE ALS TOPISCHE WIRKSTOFFE ZUR VORBEUGUNG ODER BEHANDLUNG VON PHOTODERMATOSEN**
BETA-HYDROXY KETONES AS TOPICAL AGENTS FOR THE PROPHYLAXIS OR TREATMENT OF PHOTODERMATOSES
BÊTA-HYDROXY-CÉTONE UTILISÉE COMME PRINCIPE ACTIF À USAGE TOPIQUE POUR LA PRÉVENTION OU LE TRAITEMENT DE PHOTODERMATOSES

(30) Priorität: 27.11.2015 EP 15196821
(43) Veröffentlichungstag der Anmeldung: 03.10.2018
(73) Patentinhaber: Merck Patent GmbH, 64293 Darmstadt (DE)
(72) Erfinder: CAROLA, Christophe, 64625 Bensheim (DE); HEIDER, Lilia, 64579 Gernsheim (DE); LEFORT, Marina, 64291 Darmstadt (DE); DRILLER, Hansjuergen, 64823 Gross-Umstadt (DE)
(86) Internationale Anmeldenummer: PCT/EP2016/001815
(87) Internationale Veröffentlichungsnummer: WO 2017/088950

(56) Entgegenhaltungen:
- WO-A2-2013/045016
- DE-A1-102006 019 044
- K. S. RAVIKUMAR ET AL: "Diastereoselectivity in the Reduction of Acyclic Carbonyl Compounds with Diisopropoxytitanium(III) Tetrahydroborate", THE JOURNAL OF ORGANIC CHEMISTRY, Bd. 64, Nr. 16, 1. August 1999 (1999-08-01), Seiten 5841-5844, XP055334721, ISSN: 0022-3263, DOI: 10.1021/jo9902906
- Aiguo Hu ET AL: "Supporting Information for Ru-Catalyzed Asymmetric Hydrogenation of [alpha]-Phthalimide Ketones and 1,3-Diaryl Diketones Using 4,4'-Substituted BINAPs", Organic Letters; Vol. 7, No. 3, 1. Januar 2005 (2005-01-01), XP055334019, Gefunden im Internet: URL:http://pubs.acs.org/doi/suppl/10.1021/ ol0474812 [gefunden am 2017-01-11]
- FUSARO R M ET AL: "Topical photoprotection for hereditary polymorphic light eruption of American Indians", JOURNAL OF THE AMERICAN ACADEMY OF DERMATOLOGY, MOSBY, INC, US, Bd. 24, Nr. 5, 1. Mai 1991 (1991-05-01), Seiten 744-746, XP025616630, ISSN: 0190-9622, DOI: 10.1016/0190-9622(91)70114-H [gefunden am 1991-05-01]

## Beschreibung

Die vorliegende Erfindung betrifft spezielle β-Hydroxy-Ketone der Formel (I) zur Verwendung bei der Vorbeugung und/oder Behandlung von Photodermatosen. Die speziellen β-Hydroxy-Ketonen der Formel (I) können als Wirkstoff in topischen Zusammensetzungen zur Vorbeugung und/oder nicht-therapeutischen Behandlung von Photodermatosen, insbesondere der polymorphen Lichtdermatose verwendet werden.

Photodermatosen sind Krankheiten der Haut, die unter Einwirkung von Strahlen entstehen, vorzugsweise von UVA-Strahlen, UVB-Strahlen und/oder visueller Strahlen, wobei diese Strahlen aus natürlicher Quelle oder künstlichen Quellen stammen können.

Neben dem klassischen Sonnenbrand umfassen Photodermatosen beispielsweise auch Hautveränderungen aufgrund von phototoxischer Reaktion oder photoallergischer Reaktion auf Arzneimittel oder insbesondere auch die polymorphe Lichtdermatose.

Das Erscheinungsbild der polymorphen Lichtdermatose wird in englischsprachigen Kulturen auch als polymorphic light eruption oder polymorphic light dermatose bezeichnet, oder mit den Abkürzungen PLE, PME, PMLE oder PLD versehen. Die Ursache ist unbekannt. Es wird jedoch vermutet, dass eine verspätete allergische Reaktion auftritt.

Die polymorphe Lichtdermatose ist eine sehr häufig auftretende Photodermatose, bei der man annimmt, dass insbesondere die Einwirkung von UVA-Strahlung verantwortlich ist. Ca. 11-21% Nordeuropäer sind wahrscheinlich von diesen Hauterscheinungen betroffen.

Die polymorphe Lichtdermatose tritt meist im Frühjahr oder Frühsommer nach der ersten Sonnenbestrahlung auf. Bei neuerlicher Sonnenexposition werden die Schübe im Laufe des Sommers meistens schwächer.
Die Hautveränderungen treten vor allem an den Außenseiten der Oberarme, am Halsausschnitt und auch im Gesicht auf. Die Hauterscheinungen können die unterschiedlichste Form (z.B. Rötung, Bläschen, Knötchen, nässende Hautdefekte, Hautverdickung) haben. Sie sind aber meistens bei einer Person auf eine dieser Formen beschränkt. Immer ist ein starker Juckreiz vorhanden.

Zur Vorbeugung der Ausbildung der polymorphen Lichtdermatose wird empfohlen, die direkte Sonneneinstrahlung zu vermeiden, insbesondere an Tagen und Zeiten mit hoher Lichtintensität, sowie die Benutzung von Sonnenschutzmitteln, die insbesondere UVA-Filter enthalten.

Geeignete Sonnenschutzmittel sind beispielsweise in A. Fourtanier et al, Photodermatoloty, Photoimmunology & Photomedicine 2008, 24, 164-174 beschrieben.

Gegebenenfalls enthalten derartige Sonnenschutzmittel neben den UVA-Filtern noch Antioxidantien wie beispielsweise alpha-Glucosylrutin, wie in IM Hadshiew et al, Photodermatoloty, Photoimmunology & Photomedicine 2004, 20(4), 200-4 beschrieben.

Zur Behandlung von PLE werden oft topische Zubereitungen enthaltend Corticosteroide verwendet oder es wird eine Photo(chemo)therapie durchgeführt.

Es besteht jedoch weiterhin Bedarf nach Wirkstoffen, die sich zur Vorbeugung und/oder Behandlung von Photodermatosen, insbesondere von polymorpher Lichtdermatose eignen.

Aufgabe der Erfindung ist es daher, Wirkstoffe zur Verfügung zu stellen, die eine therapeutische und/oder nicht-therapeutische Vorbeugung und/oder Behandlung von Photodermatosen, insbesondere von polymorpher Lichtdermatose ermöglichen.

Überraschenderweise wurde gefunden, dass spezielle β-Hydroxy-Ketone der Formel (I) diese Aufgabe lösen.

Ein erster Gegenstand der Erfindung sind daher Verbindungen der Formel (I) 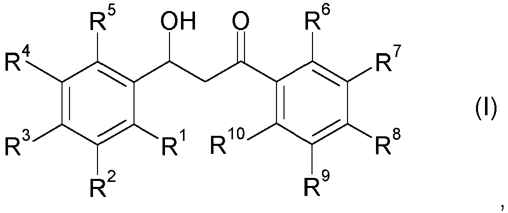 wobei
R¹ bis R¹⁰ jeweils unabhängig voneinander H, geradkettige oder verzweigte C₁- bis C₂₀-Alkylgruppe, geradkettige oder verzweigte C₁- bis C₂₀-Alkoxygruppe oder geradkettige oder verzweigte C₁- bis C₂₀-Dialkylaminogruppe bedeuten, als Solches wirksam
zur Verwendung bei der Vorbeugung und/oder Behandlung von polymorpher Lichtdermatose.

Weiter beschrieben ist die Verwendung einer Verbindung der Formel (I) 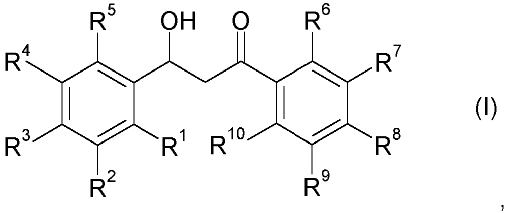 wobei

R¹ bis R¹⁰ jeweils unabhängig voneinander H, geradkettige oder verzweigte C₁- bis C₂₀-Alkylgruppe, geradkettige oder verzweigte C₁- bis C₂₀-Alkoxygruppe oder geradkettige oder verzweigte C₁- bis C₂₀-Dialkylaminogruppe bedeuten, als Wirkstoff in einer kosmetischen Zubereitung zur nicht-therapeutischen Behandlung und/oder Vorbeugung einer Photodermatose, insbesondere zur nicht-therapeutischen Behandlung und/oder Vorbeugung von polymorpher Lichtdermatose.

Die Verbindungen der Formel (I), wie zuvor beschrieben, sind unter anderem aus WO 2007/121845 bekannt. Die Beschreibung der WO 2007/121845 umfasst weitere Verbindungen, die nicht unter die Formel (I) fallen, wie zuvor beschrieben. Generell sind alle aus WO 2007/121845 bekannten Verbindungen als Antioxidantien beschrieben. Wie dort unter Bezugnahme auf CD Römpp Chemie Lexikon - Version 1.0, Stuttgart/New York: Georg Thieme Verlag 1995 beschrieben, handelt es sich bei Antioxidantien um Verbindungen, die unerwünschte, durch Sauerstoff-Einwirkungen u.a. oxidative Prozesse bedingte Veränderungen in den zu schützenden Stoffen hemmen oder verhindern. Sie wirken daher insbesondere gegen freie Radikale, die durch UV-Strahlung auf der Haut entstehen. WO 2007/121845 beschreibt weiterhin, dass spezielle Verbindungen aus der Offenbarung auch gegen eine Vielzahl von Hautkrankheiten wirken können, ohne jedoch das Erscheinungsbild der Photodermatose oder insbesondere der polymorphen Lichtdermatose explizit zu beschreiben.

Es handelt sich daher bei der vorliegenden Erfindung um eine zweite medizinische Verwendung bekannter Verbindungen.

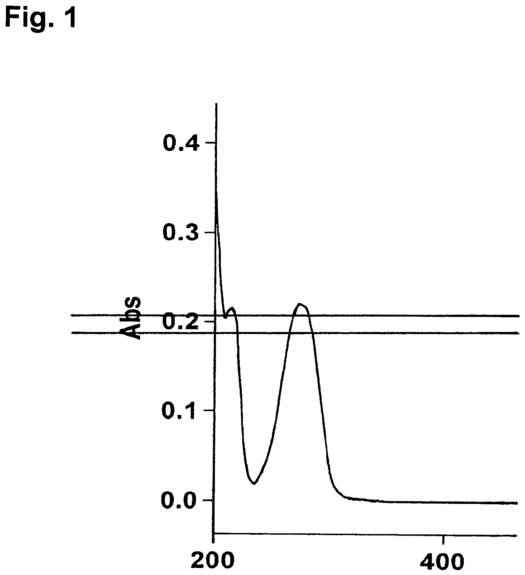

Die Verbindungen der Formel (I) 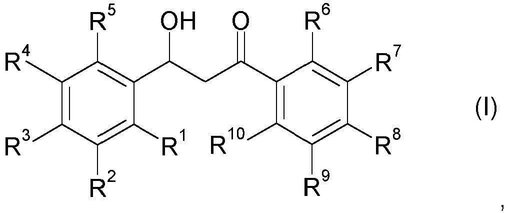 wobei
R¹ bis R¹⁰ jeweils unabhängig voneinander H, geradkettige oder verzweigte C₁- bis C₂₀-Alkylgruppe, geradkettige oder verzweigte C₁- bis C₂₀-Alkoxygruppe oder geradkettige oder verzweigte C₁- bis C₂₀-Dialkylaminogruppe bedeuten, sind keine UV-Filter, d.h. sie zeigen keine UV-Absorption. Das Absorptionsverhalten ist stellvertretend für die Verbindung 3-(4-tert-Butyl-phenyl)-3-hydroxy-1-(4-methoxy-phenyl)-propan-1-on in Figur 1 dargestellt. Bei Wellenlängen von 273 nm und 215,5 nm liegt die Absorption lediglich bei 0.22, d.h. die Verbindung 3-(4-tert-Butylphenyl)-3-hydroxy-1-(4-methoxy-phenyl)-propan-1-on absorbiert nicht im UVA-Bereich (UVA, 320-400 nm).

Es ist weiterhin bekannt, dass Verbindungen der Formel (I), wie zuvor beschrieben oder später als bevorzugt beschrieben, außerdem photostabilisierend auf die entsprechenden korrespondierenden Diketonderivate wirken. Die Einwirkung von Sonnenlicht löst die Umwandlung der Verbindungen der Formel (I) in ihre Diketonderivate aus, die dann in der Lage sind, als UVA-Absorber zu agieren. In Sonnenschutzformulierungen ermöglicht dies somit die gezielte Bereitstellung der entsprechenden UVA-Absorber ausgehend von Verbindungen der Formel (I) bei Sonneneinstrahlung. Diese Photokonversion muss jedoch beschleunigt werden, wenn ein signifikanter Anteil an UVA-Filter erzeugt werden soll. Dies belegen die Offenlegungsschriften WO 2011/141111 und DE 102012016960 A1. Stellvertretend für die Verbindungen der Formel (I) wird im ausführenden Teil die Wirksamkeit von 3-(4-tert-Butyl-phenyl)-3-hydroxy-1-(4-methoxyphenyl)-propan-1-on *in vivo* belegt, wobei unter den gewählten Bedingungen der Studie der Anteil an entstehendem UVA-Filter durch Zerfall von 3-(4-tert-Butyl-phenyl)-3-hydroxy-1-(4-methoxy-phenyl)-propan-1-on unter Einwirkung von UV-Licht lediglich 0.1 Gew.% beträgt. Ein derartiger Anteil eines UVA-Filters bewirkt keinen effizienten UVA-Schutz und kann daher nicht für die positiven Ergebnisse der *in vivo* Studie und der Wirksamkeit gegen eine Ausbildung einer Photodermatose, insbesondere einer polymorphen Lichtdermatose, verantwortlich sein.

Die Verbindungen der Formel (I) verhindern demzufolge das Auftreten des Erscheinungsbilds einer Photodermatose, insbesondere der polymorphen Lichtdermatose. Demzufolge ist die zweite medizinische bzw. die zweite nicht-medizinische Verwendung der Verbindungen der Formel (I) bevorzugt eine Vorbeugung von Photodermatosen, besonders bevorzugt eine Vorbeugung von polymorpher Lichtdermatose.

Darüber hinaus ist es sogar von Vorteil, dass die als Solches wirksamen Verbindungen der Formel (I), wie zuvor beschrieben und nachfolgend bevorzugt beschrieben, sich nach Zerfall durch Einwirkung von Sonnenlicht in einen UVA-Filter umwandeln können, und so selbst nach eigenem Zerfall durch Sonneneinwirkung, eine Prävention der Photodermatose weiter unterstützen.

Es wird weiter vermutet, dass die Verbindungen der Formel (I), wie zuvor beschrieben oder nachfolgend bevorzugt beschrieben, eine entzündungshemmende Wirkung aufweisen. Die Verbindungen der Formel (I), insbesondere 3-(4-tert-Butyl-phenyl)-3-hydroxy-1-(4-methoxy-phenyl)-propan-1-on, inhibieren/inhibiert die Aktivierung des Transkriptionsfaktors NF-κB,
welcher ein Schlüsselregulator bei der Expression von vielen Genen ist, die bei einer Entzündung involviert sind, wie in Baeuerle, Cell. 1998, 95(6):729-31 beschrieben.

Wie zuvor beschrieben, können die Substituenten R¹ bis R¹⁰ jeweils unabhängig voneinander H, geradkettige oder verzweigte C₁- bis C₂₀-Alkylgruppe, geradkettige oder verzweigte C₁- bis C₂₀-Alkoxygruppe oder geradkettige oder verzweigte C₁- bis C₂₀-Dialkylaminogruppe bedeuten.

Geradkettige oder verzweigte Alkylgruppen mit 1 bis 4, 1 bis 8, 1 bis 12 oder 1 bis 20 C-Atomen entsprechen der Formel CₚH₂ₚ₊₁ mit p = 1, 2, 3 oder 4, oder 1, 2, 3, 4, 5, 6, 7 oder 8, oder 1, 2, 3, 4, 5, 6, 7, 8, 9, 10, 11 oder 12, oder 1, 2, 3, 4, 5, 6, 7, 8, 9, 10, 11, 12, 13, 14, 15, 16, 17, 18, 19 oder 20, beispielsweise Methyl, Ethyl, i-Propyl, Propyl, Butyl, i-Butyl oder tert-Butyl, Pentyl, 1-, 2- oder 3-Methylbutyl, 1,1-, 1,2- oder 2,2-Dimethylpropyl, 1-Ethylpropyl oder Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl oder Eicosyl.
Wird eine Alkylgruppe nicht näher bezeichnet, so handelt es sich um eine geradkettige Alkylgruppe.

Geradkettige oder verzweigte Alkoxygruppen mit 1 bis 4, 1 bis 8, 1 bis 12 oder 1 bis 20 C-Atomen entsprechen der Formel OCₚH₂ₚ₊₁ mit p = 1, 2, 3 oder 4, oder 1, 2, 3, 4, 5, 6, 7 oder 8, oder 1, 2, 3, 4, 5, 6, 7, 8, 9, 10, 11 oder 12, oder 1, 2, 3, 4, 5, 6, 7, 8, 9, 10, 11, 12, 13, 14, 15, 16, 17, 18, 19 oder 20, beispielsweise Methoxy, Ethyoxy, i-Propyloxy, Propyloxy, Butyloxy, i-Butyloxy oder tert-Butyloxy, Pentyloxy, 1-, 2- oder 3-Methylbutyloxy, 1,1-, 1,2- oder 2,2-Dimethylpropyloxy, 1-Ethylpropyloxy oder Hexyloxy, Heptyloxy, Octyloxy, Nonyloxy, Decyloxy, Undecyloxy, Dodecyloxy, Tridecyloxy, Tetradecyloxy, Pentadecyloxy, Hexadecyloxy, Heptadecyloxy, Octadecyloxy, Nonadecyloxy oder Eicosyloxy. Geradkettige oder verzweigte Alkoxygruppen mit 1 bis 4, 1 bis 8, 1 bis 12 oder 1 bis 20 C-Atomen entsprechen der Formel OCₚH₂ₚ₊₁ mit p = 1, 2, 3 oder 4, oder 1, 2, 3, 4, 5, 6, 7 oder 8, oder 1, 2, 3, 4, 5, 6, 7, 8, 9, 10, 11 oder 12, oder 1, 2, 3, 4, 5, 6, 7, 8, 9, 10, 11, 12, 13, 14, 15, 16, 17, 18, 19 oder 20, beispielsweise Methoxy, Ethyoxy, i-Propyloxy, Propyloxy, Butyloxy, i-Butyloxy oder tert-Butyloxy, Pentyloxy, 1-, 2- oder 3-Methylbutyloxy, 1,1-, 1,2- oder 2,2-Dimethylpropyloxy, 1-Ethylpropyloxy oder Hexyloxy, Heptyloxy, Octyloxy, Nonyloxy, Decyloxy, Undecyloxy, Dodecyloxy, Tridecyloxy, Tetradecyloxy, Pentadecyloxy, Hexadecyloxy, Heptadecyloxy, Octadecyloxy, Nonadecyloxy oder Eicosyloxy.

Geradkettige oder verzweigte Dialkylaminogruppen mit 1 bis 4, 1 bis 8, 1 bis 12 oder 1 bis 20 C-Atomen entsprechen der Formel N(CₚH₂ₚ₊₁)₂ mit p jeweils unabhängig voneinander 1, 2, 3 oder 4, oder 1, 2, 3, 4, 5, 6, 7 oder 8, oder 1, 2, 3, 4, 5, 6, 7, 8, 9, 10, 11 oder 12, oder 1, 2, 3, 4, 5, 6, 7, 8, 9, 10, 11, 12, 13, 14, 15, 16, 17, 18, 19 oder 20, beispielsweise Dimethylamino, Methylethylamino, Diethylamino, Di(i-Propyl)amino, Methylpropylamino, Ethylpropylamino, Dipropylamino, Methylbutylamino, Ethylbutylamino, Propylbutylamino, Dibutylamino, Di(i-Butyl)amino oder Di(tert-Butyl)amino, Dipentylamino, Di(1-, 2- oder 3-Methyl)butylamino, Di(1,1-, 1,2- oder 2,2-Dimethylpropyl)amino, Di(1-Ethylpropyl)amino oder Dihexylamino, Diheptylamino, Dioctylamino, Dinonylamino, Didecylamino, Diundecylamino, Didodecylamino, Ditridecylamino, Ditetradecylamino, Dipentadecylamino, Dihexadecylaminoy, Diheptadecylamino, Dioctadecylamino, Dinonadecylamino oder Dieicosylamino.

In den Substituenten R¹ bis R¹⁰ sind die Alkylgruppen jeweils unabhängig voneinander geradkettig oder verzweigt und haben bevorzugt 1 bis 12 C-Atome, besonders bevorzugt 1 bis 4 C-Atome.
In den Substituenten R¹ bis R¹⁰ sind die Alkoxygruppen jeweils unabhängig voneinander geradkettig oder verzweigt und haben bevorzugt 1 bis 12 C-Atome, besonders bevorzugt 1 bis 4 C-Atome.

In den Substituenten R¹ bis R¹⁰ sind die Diaminoalkylgruppen jeweils unabhängig voneinander geradkettig oder verzweigt und haben bevorzugt 1 bis 12 C-Atome, besonders bevorzugt 1 bis 4 C-Atome.

Bevorzugte Verbindungen der Formel (I) sind Verbindungen, in denen R¹, R², R⁴, R⁵, R⁶, R⁷, R⁹ und R¹⁰ H bedeuten und R³ und R⁸ jeweils unabhängig voneinander geradkettige oder verzweigte C₁- bis C₁₂-Alkylgruppe oder geradkettige oder verzweigte C₁- bis C₁₂-Alkoxygruppe bedeuten.

Besonders bevorzugte Verbindungen der Formel (I) sind Verbindungen, in denen R¹, R², R⁴, R⁵, R⁶, R⁷, R⁹ und R¹⁰ H bedeuten und R³ und R⁸ jeweils unabhängig voneinander geradkettige oder verzweigte C₁- bis C₄-Alkylgruppe oder geradkettige oder verzweigte C₁- bis C₄-Alkoxygruppe bedeuten.

Ganz besonders bevorzugte Verbindungen der Formel (I) sind 3-(4-tert-Butyl-phenyl)-3-hydroxy-1-(4-methoxy-phenyl)-propan-1-on, 1-(4-tert-Butylphenyl)-3-hydroxy-3-(4-methoxy-phenyl)-propan-1-on oder deren Mischung. Ganz besonders bevorzugt wird die Verbindung 3-(4-tert-Butylphenyl)-3-hydroxy-1-(4-methoxy-phenyl)-propan-1-on erfindungsgemäß verwendet.

Ein weiterer Gegenstand der Erfindung sind daher Verbindungen der Formel (I), wie zuvor beschrieben, wobei R¹, R², R⁴, R⁵, R⁶, R⁷, R⁹ und R¹⁰ H bedeuten und R³ und R⁸ jeweils unabhängig voneinander geradkettige oder verzweigte C₁- bis C₁₂-Alkylgruppe oder geradkettige oder verzweigte C₁- bis C₁₂-Alkoxygruppe bedeuten, als Solches wirksam, zur Verwendung bei der Vorbeugung und/oder Behandlung von polymorpher Lichtdermatose.

Ein weiterer Gegenstand der Erfindung sind daher Verbindungen der Formel (I), wie zuvor beschrieben, wobei R¹, R², R⁴, R⁵, R⁶, R⁷, R⁹ und R¹⁰ H bedeuten und R³ und R⁸ jeweils unabhängig voneinander geradkettige oder verzweigte C₁- bis C₄-Alkylgruppe oder geradkettige oder verzweigte C₁- bis C₄-Alkoxygruppe bedeuten, als Solches wirksam, zur Verwendung bei der Vorbeugung und/oder Behandlung von polymorpher Lichtdermatose.

Ein weiterer Gegenstand der Erfindung sind daher die Verbindungen 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder deren Mischung, als Solches wirksam, zur Verwendung bei der Vorbeugung und/oder Behandlung von polymorpher Lichtdermatose.

Weiterer beschrieben ist die Verwendung von Verbindungen der Formel (I) als Wirkstoff in einer kosmetischen Zubereitung zur nicht-therapeutischen Behandlung und/oder Vorbeugung einer Photodermatose, insbesondere zur Vorbeugung einer polymorphen Lichtdermatose, wobei die Substituenten R¹, R², R⁴, R⁵, R⁶, R⁷, R⁹ und R¹⁰ der Verbindung der Formel (I) H bedeuten und R³ und R⁸ jeweils unabhängig voneinander geradkettige oder verzweigte C₁- bis C₁₂-Alkylgruppe oder geradkettige oder verzweigte C₁- bis C₁₂-Alkoxygruppe bedeuten.

Weiter beschrieben ist die Verwendung von Verbindungen der Formel (I) als Wirkstoff in einer kosmetischen Zubereitung zur nicht-therapeutischen Behandlung und/oder Vorbeugung einer Photodermatose, insbesondere zur Vorbeugung einer polymorphen Lichtdermatose, wobei die Substituenten R¹, R², R⁴, R⁵, R⁶, R⁷, R⁹ und R¹⁰ der Verbindung der Formel (I) H bedeuten und R³ und R⁸ jeweils unabhängig voneinander geradkettige oder verzweigte C₁- bis C₄-

Alkylgruppe oder geradkettige oder verzweigte C₁- bis C₄-Alkoxygruppe bedeuten.

Weiter beschrieben ist die Verwendung der Verbindungen 3-(4-tert-Butylphenyl)-3-hydroxy-1-(4-methoxy-phenyl)-propan-1-on, 1-(4-tert-Butylphenyl)-3-hydroxy-3-(4-methoxy-phenyl)-propan-1-on oder deren Mischung als Wirkstoff(e) in einer kosmetischen Zubereitung zur nicht-therapeutischen Behandlung und/oder Vorbeugung einer Photodermatose, insbesondere zur Vorbeugung einer polymorphen Lichtdermatose.

Weiter beschrieben ist ein nicht-therapeutisches Verfahren zur Vorbeugung von Photodermatosen, wobei auf die Haut eine kosmetische Zubereitung aufgetragen wird, die mindestens eine Verbindung der Formel (I), wie zuvor beschrieben oder als bevorzugt beschrieben, enthält.

Weiter beschrieben ist ein nicht-therapeutisches Verfahren zur Vorbeugung von polymorpher Lichtdermatose, wobei auf die Haut eine kosmetische Zubereitung aufgetragen wird, die 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder deren Mischung, enthält.

Die Art der kosmetischen oder pharmazeutischen Zubereitung enthaltend mindestens eine Verbindung der Formel (I), wie zuvor beschrieben oder bevorzugt beschrieben, ist hierbei nicht eingeschränkt.

Geeignete Zubereitungen sind beispielsweise aus WO 2007/121845, WO 2011/141111 und DE 102012016960 A1 bekannt.

Die Verbindungen der Formel (I), wie zuvor beschrieben oder bevorzugt beschrieben, werden in der zweiten nicht-medizinischen Verwendung bzw. in der zweiten medizinischen Verwendung bevorzugt in einer Menge von 1 bis 10 Gewichtsprozent, vorzugsweise 2 - 5 Gew.-%, in die entsprechenden Zubereitungen eingearbeitet Eine zweite nicht-medizinische Verwendung ist nicht beansprucht und entsprechend nicht erfindungsgemäß.

Vorteilhaft ist eine Kombination der Verbindungen der Formel (I), wie zuvor beschrieben oder als bevorzugt beschrieben, mit anderen Wirkstoffen, die ebenfalls vorbeugend oder behandelnd für Photodermatosen, bevorzugt für polymorphe Lichtdermatose, sind.

Besonders geeignete Wirkstoffe zur Kombination sind beispielsweise 1-(4-Methoxyphenyl)-3-(4-tert-butylphenyl)-1,3-propandion (Eusolex® 9020 (Firma Merck)), Escamule (Mexoryl® SX (der Firma Chimex), Breitbandfilter wie unbeschichtete oder beschichtete Titandioxide, Zinkoxide oder partikuläre Filter wie Tinosorb® S, Tinosorb® M oder Tris Biphenyl Triazine (TBPT), vertrieben von der Firma BASF, gegebenenfalls in Kombination mit geeigneten Antioxidantien wie beispielsweise α-Glucosylrutin, Isoquercetin oder 2-(4-Hydroxy-3,5-dimethoxybenzy)-malonsäure-bis-(2-ethylhexyl)ester (RonaCare® AP (Firma Merck)).

Die folgenden Ausführungsbeispiele erläutern die Erfindung.

### Beispiele:

Die Verbindung 3-(4-tert-Butyl-phenyl)-3-hydroxy-1-(4-methoxy-phenyl)-propan-1-on (abgekürzt Verbindung A) wird analog zu der Beschreibung der DE 102010055084 A1, Beispiel 1 hergestellt.

In der *in vivo* Studie, die im Folgenden beschrieben wird, wird folgende O/W-Emulsion verwendet:

| Inhaltsstoffe | INCI | [Gew.%] | [g] |
|---|---|---|---|
| **A** | | | |
| Verbindung A | Methoxy Monobenzoylmethane | 2,0 | 20,0 |
| Montanov 202 | Arachidyl Alcohol, Behenyl Alcohol | 3,0 | 30,0 |
| Lanol 99 | Isononyl Isononanoate | 2,0 | 20,0 |
| Miglyol 812N | Caprylylic/Capric Triglyceride | 2,0 | 20,0 |
| Cetiol CC | Dicaprylyl Carbonate | 3,0 | 30,0 |
| Cocoate BG | Butylene Glycol Cocoate | 2,0 | 20,0 |

| **B** | | | |
|---|---|---|---|
| Wasser, demin. | Aqua (water) | 76,7 | 767,0 |
| 1,2-Propanediol | Propylene Glycol | 6,0 | 60,0 |
| Phospholipon 90 G | Phosphatidyl Choline | 0,3 | 3,0 |

| **C** | | | |
|---|---|---|---|
| Simulgel NS | Hydroxyethyl Acrylate/Sodium, Squalane, Polysorbate 60, Acryloyldimethyltaurate Copolymer | 2,β | 20,0 |

| **E** | | pH 5,9 | |
|---|---|---|---|
| Euxyl PE9010 | Phenoxyethanol, Ethylhexylglycerin | 1,0 | 10,0 |
| Zitronensäure | Aqua(water) Citric Acid | 1 Tropfen | |

Es wird eine Vergleichsstudie mit Positivkontrolle an 6 Probanden mit bekanntem PLE Syndrom durchgeführt. Die Studie wurde in Deutschland im April über einen Zeitraum von 5 Tagen durchgeführt. Die Positivkontrolle war unbehandelte, bestrahlte Haut. Es wurde mit 100% UVA-Licht bestrahlt, in einer sub-erythemalen Dosis, welche individuell bestimmt wurde. Die Zubereitung wurde täglich auf die Hälfte des Dekolletees aufgetragen (2mg/cm²).
Die Messungen wurde zwei Mal am Tag durchgeführt, 10 min. nach Auftrag der Zubereitung und direkt nach Bestrahlung.

Unter den Bedingungen der *in vivo* Studie kann ausgeschlossen werden, dass eine Konversion der Verbindung A zu dem UVA-Filter Avobenzon in einer Menge erfolgt, die Einfluss auf die Hauterscheinungen der Probanden ausüben könnte.

Bestrahlt man nämlich eine 2%ige Lösung der Verbindung A in Cosmacol® ELI (C12-13 Alkyl Lactate) mit UVA/UVB-Licht (20/1) in einer Dosierung von 5 MED (entsprechend 250kJ/m²), so entsteht 0.1% Avobenzon durch Konversion.
Unter den Bedingungen des *in vivo* Experiments, wird eine UVA Dosis von 20J/cm² (entsprechend 200kJ/m²) bestrahlt, d.h. unter diesen Bedingungen wird sogar weniger als 0.1% Avobenzon durch Konversion entstehen.

**Tabelle 1 zeigt Hautreaktionen unter UVA-Bestrahlung bei unbehandelter Haut:**

| Tag | Bestrahlung | Proband | | | | | |
|---|---|---|---|---|---|---|---|
| | | 1 | 2 | 3 | 4 | 5 | 6 |
| 1 | vorher | 0 | 0 | 0 | 0 | 0 | 0 |
| | nachher | 0 | 0 | 0 | 0 | 0 | 0 |
| 2 | vorher | 0 | ± | ± | 0 | 0 | 0 |
| | nachher | Juckreiz | ± | ± | 0 | 0 | 0 |
| 3 | vorher | ± | ± | ± | ± | 0 | ± |
| | nachher | ± | ± | ± | ± | 0 | + |
| 4 | vorher | ± | ± | ± | ± | 0 | + |
| | nachher | + | + | ± | + | + | + |
| 5 | vorher | + | + | + | + | + | + |
| | nachher | + | + | + | + | + | + |

**Tabelle 2 zeigt Hautreaktionen unter UVA-Bestrahlung bei behandelter Haut:**

| Tag | Bestrahlung | Proband | | | | | |
|---|---|---|---|---|---|---|---|
| | | 1 | 2 | 3 | 4 | 5 | 6 |
| 1 | vorher | 0 | 0 | 0 | 0 | 0 | 0 |
| | nachher | 0 | 0 | 0 | 0 | 0 | 0 |
| 2 | vorher | 0 | 0 | 0 | 0 | 0 | 0 |
| | nachher | 0 | 0 | 0 | 0 | 0 | 0 |
| 3 | vorher | 0 | 0 | 0 | 0 | 0 | ± |
| | nachher | 0 | 0 | 0 | 0 | 0 | ± |
| 4 | vorher | ± | 0 | 0 | 0 | 0 | ± |
| | nachher | ± | 0 | 0 | 0 | 0 | ± |
| 5 | vorher | ± | 0 | 0 | 0 | 0 | ± |
| | nachher | ± | 0 | 0 | 0 | 0 | ± |

| | | | | | | | |
|---|---|---|---|---|---|---|---|
| 0 bedeutet keine Reaktion ± bedeutet schwache Reaktion + bedeutet klare Reaktion ++ bedeutet starke Reaktion +++ bedeutet sehr starke Reaktion | | | | | | | |

### Ergebnisse:

Bei allen Probanden traten Hauterscheinungen auf unbehandelter Haut auf, die das Erscheinungsbild der polymorphen Lichtdermatose haben. Bei 4 Probanden wurde keine polymorphe Lichtdermatose auf behandelter Haut beobachtet. Lediglich bei 2 Probanden wurde eine schwache Reaktion festgestellt. Die Ergebnisse belegen daher, dass die Verbindung A als Solches bei Bestrahlung die Hauterscheinungen einer polymorphen Lichtdermatose unterbindet oder signifikant reduziert.

### Verzeichnis der Figuren

Figur 1 zeigt das Absorptionsspektrum der Verbindung 3-(4-tert-Butylphenyl)-3-hydroxy-1-(4-methoxy-phenyl)-propan-1-on.

## Patentansprüche

1. Verbindungen der Formel (I), 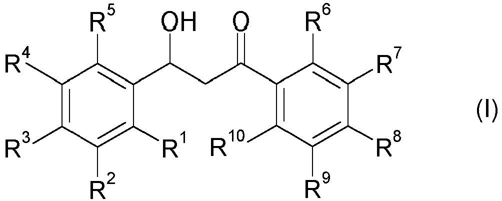 wobei
R¹ bis R¹⁰ jeweils unabhängig voneinander H, geradkettige oder verzweigte C₁- bis C₂₀-Alkylgruppe, geradkettige oder verzweigte C₁-bis C₂₀-Alkoxygruppe oder geradkettige oder verzweigte C₁- bis C₂₀-Dialkylaminogruppe bedeuten, als Solches wirksam, zur Verwendung bei der Vorbeugung und/oder Behandlung von polymorpher Lichtdermatose.

2. Verbindungen der Formel (I) nach Anspruch 1, als Solches wirksam, zur Verwendung bei der Vorbeugung und/oder Behandlung von polymorpher Lichtdermatose, wobei R¹, R², R⁴, R⁵, R⁶, R⁷, R⁹ und R¹⁰ H bedeuten und R³ und R⁸ jeweils unabhängig voneinander geradkettige oder verzweigte C₁- bis C₁₂-Alkylgruppe oder geradkettige oder verzweigte C₁- bis C₁₂-Alkoxygruppe bedeuten.

3. Verbindungen der Formel (I) nach Anspruch 1, als Solches wirksam, zur Verwendung bei der Vorbeugung und/oder Behandlung von polymorpher Lichtdermatose, wobei R¹, R², R⁴, R⁵, R⁶, R⁷, R⁹ und R¹⁰ H bedeuten und R³ und R⁸ jeweils unabhängig voneinander geradkettige oder verzweigte C₁- bis C₄-Alkylgruppe oder geradkettige oder verzweigte C₁- bis C₄-Alkoxygruppe bedeuten.

4. 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder deren Mischung, als Solches wirksam, zur Verwendung bei der Vorbeugung und/oder Behandlung von polymorpher Lichtdermatose.

5. 3-(4-tert-Butyl-phenyl)-3-hydroxy-1-(4-methoxy-phenyl)-propan-1-on, als Solches wirksam, zur Verwendung bei der Vorbeugung und/oder Behandlung von polymorpher Lichtdermatose.

## Claims

1. Compounds of the formula (I), 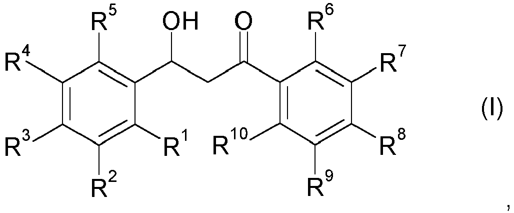 where
R¹ to R¹⁰ each, independently of one another, denote H, a straight-chain or branched C₁- to C₂₀-alkyl group, a straight-chain or branched C₁- to C₂₀-alkoxy group or a straight-chain or branched C₁- to C₂₀-dialkylamino group, effective as such, for use in the prevention and/or treatment of polymorphic photodermatosis.

2. Compounds of the formula (I) according to Claim 1, effective as such, for use in the prevention and/or treatment of polymorphic photodermatosis, where R¹, R², R⁴, R⁵, R⁶, R⁷, R⁹ and R¹⁰ denote H and R³ and R⁸ each, independently of one another, denote a straight-chain or branched C₁- to C₁₂-alkyl group or a straight-chain or branched C₁- to C₁₂-alkoxy group.

3. Compounds of the formula (I) according to Claim 1, effective as such, for use in the prevention and/or treatment of polymorphic photodermatosis, where R¹, R², R⁴, R⁵, R⁶, R⁷, R⁹ and R¹⁰ denote H and R³ and R⁸ each, independently of one another, denote a straight-chain or branched C₁- to C₄-alkyl group or a straight-chain or branched C₁- to C₄-alkoxy group.

4. 3-(4-tert-Butylphenyl)-3-hydroxy-1-(4-methoxyphenyl)propan-1-one, 1-(4-tert-butylphenyl)-3-hydroxy-3-(4-methoxyphenyl)propan-1-one or a mixture thereof, effective as such, for use in the prevention and/or treatment of polymorphic photodermatosis.

5. 3-(4-tert-Butylphenyl)-3-hydroxy-1-(4-methoxyphenyl)propan-1-one, effective as such, for use in the prevention and/or treatment of polymorphic photodermatosis.

## Revendications

1. Composés de formule (I), 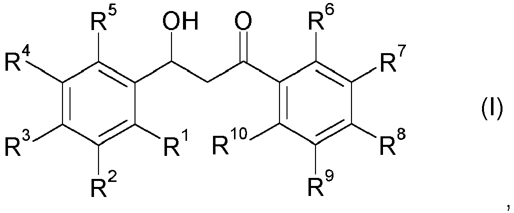 dans lesquels
R¹ à R¹⁰ désignent chacun, indépendamment les uns des autres, H, un groupement C₁- à C₂₀-alkyle à chaîne linéaire ou ramifiée, un groupement C₁- à C₂₀-alcoxy à chaîne linéaire ou ramifiée ou un groupement C₁- à C₂₀-dialkylamino à chaîne linéaire ou ramifiée, efficaces en tant que tels, pour une utilisation dans la prévention et/ou le traitement de la photodermatose polymorphe.

2. Composés de formule (I) selon la revendication 1, efficaces en tant que tels, pour une utilisation dans la prévention et/ou le traitement de la photodermatose polymorphe, dans lesquels R¹, R², R⁴, R⁵, R⁶, R⁷, R⁹ et R¹⁰ désignent H et R³ et R⁸ désignent chacun, indépendamment l'un de l'autre, un groupement C₁- à C₁₂-alkyle à chaîne linéaire ou ramifiée ou un groupement C₁- à C₁₂-alcoxy à chaîne linéaire ou ramifiée.

3. Composés de formule (I) selon la revendication 1, efficaces en tant que tels, pour une utilisation dans la prévention et/ou le traitement de la photodermatose polymorphe, dans lesquels R¹, R², R⁴, R⁵, R⁶, R⁷, R⁹ et R¹⁰ désignent H et R³ et R⁸ désignent chacun, indépendamment l'un de l'autre, un groupement C₁- à C₄-alkyle à chaîne linéaire ou ramifiée ou un groupement C₁- à C₄-alcoxy à chaîne linéaire ou ramifiée.

4. 3-(4-tertio-Butylphényl)-3-hydroxy-1-(4-méthoxyphényl)propan-1-one, 1-(4-tertio-butylphényl)-3-hydroxy-3-(4-méthoxyphényl)propan-1-one ou un mélange de celles-ci, efficaces en tant que telles, pour une utilisation dans la prévention et/ou le traitement de la photodermatose polymorphe.

5. 3-(4-tertio-Butylphényl)-3-hydroxy-1-(4-méthoxyphényl)propan-1-one, efficace en tant que telle, pour une utilisation dans la prévention et/ou le traitement de la photodermatose polymorphe.
